# EUROPEAN PATENT SPECIFICATION

(11) **EP 0 428 223 B1**
(45) Date of publication and mention of the grant of the patent: **06.07.1994**
(21) Application number: 90202989.1
(22) Date of filing: 12.11.1990
(51) Int. Cl.: B01J 21/06, B01J 37/00, C07C 1/04

(54) **Process for the preparation of extrudates**
Verfahren zur Herstellung von Extrudaten
Procédé de préparation d'extrudats

(30) Priority: 16.11.1989 GB 8925979
(43) Date of publication of application: 22.05.1991
(73) Proprietor: SHELL INTERNATIONALE RESEARCH MAATSCHAPPIJ B.V., 2596 HR Den Haag (NL)
(72) Inventor: Reinalda, Donald, NL-1031 CM Amsterdam (NL); Derking, Anke, NL-1031 CM Amsterdam (NL); Blankenstein, Paul, NL-1031 CM Amsterdam (NL); Meuris, Theofiel, B-9000 Gent (BE); Decleer, Jos Gerard Monica, B-9000 Gent (BE)

(56) References cited:
- EP-A- 0 167 324
- EP-A- 0 309 048
- FR-A- 2 590 887
- GB-A- 2 050 858
- US-A- 3 067 127

## Description

The invention relates to a process for the preparation of extrudates of use in the manufacture of catalyst carriers and/or catalysts especially useful as Fischer-Tropsch catalysts.

The preparation of hydrocarbons from gas mixtures comprising hydrogen and carbon monoxide by contacting these mixtures with a suitable catalyst at elevated temperature and pressure is known in the literature as the Fischer-Tropsch synthesis.

Catalysts often used for this purpose comprise one or more metals from group VIII of the Periodic Table, especially from the iron group, supported on a carrier, optionally in combination with one or more metal oxides and/or other metals as promoters. The metal oxide promoters are usually chosen from groups IIa, IIIb, IVb, and/or Vb of the Periodic Table as well as from the lanthanides and/or actinides. The metal promoter may be selected from the groups VIIb and/or VIII of the Periodic Table.

Very suitable Fischer-Tropsch catalysts, especially catalysts containing cobalt and zirconium on silica carriers, have been described in the literature, see for instance European patent application No. 127220.

The Fischer-Tropsch catalysts as indicated above are usually prepared by combining a carrier and one or more suitable metal compounds, e.g. by precipitating the metals, either separately or together, on the carrier from one or more solutions, or, preferably, by impregnating the carrier with compounds of the metals dissolved in a liquid in one or more steps. Moreover, kneading techniques may be applied as well. In all preparation procedures solvent is usually removed by evaporation from the products obtained, followed by calcination of the dried products. Thereafter, the calcined product is activated, usually by reduction with a hydrogen containing gas.

It has been observed that, when using silica as carrier for the preparation of cobalt based Fischer-Tropsch catalysts, in some cases cobalt hydrosilicates are formed. These cobalt hydrosilicates do not exhibit any significantly catalytic activity, while reduction of these hydrosilicates into catalytically active cobalt metal is laborious and requires severe conditions. Thus, it has been proposed to apply first another metal oxide on the carrier, especially zirconia in view of its positive effects on the activity, stability and selectivity on cobalt based Fischer-Tropsch catalysts, followed by the application of cobalt. As this implies several impregnation steps, extensive research has been carried out in order to develop a simple process for the manufacture of porous catalyst carriers, preferably silica, at least partially coated with one or more other metal oxides, especially with sirconia, for use in the preparation of catalysts or catalyst precursors. The research has been especially directed to the development of extruded carriers, for example silica carriers, in view of the problems encountered when using more or less spherical particles, for example silica spheres, such as high pressure drops, inhomogeneous distributions (diameter, pore diameter) and heat transfer problems. Further, the ratio of the external surface area and volume is more favourable in the case of extrudates, especially trilobes or other polylobal particles, than in the case of spheres.

A method for the preparation of silica extrudates wherein a particulate silica is mixed with water and an alkali metal compound, followed by mulling and extruding the mixture and subsequently drying the extrudate, is disclosed in European patent application No. 167324. However, the use of alkali metal compounds makes it very often necessary that the silica extrudates have to be soaked in a solution of ammonium nitrate and nitric acid to remove the alkali metal ions as the presence of alkali metal ions often impairs the catalytic performance of the extrudates. Other known processes for the extrusion of silica result in products having a low crush strength only.

European patent application publication No. 309048 discloses a process for the preparation of silica extrudates which comprises mixing and kneading a particulate silica with water and with ammonia or an ammonia-releasing compound to obtain a mixture having a total solids content of from 25 to 60% by weight, the ammonia being present in an amount of from 0.5 to 20% by weight on the total solids content of the mixture and extruding the mixture. It is possible prior to extrusion to admix titanium dioxide or zirconium dioxide to the composition.

The extrudates described in the said patent application do not solve the problem the present invention sets out to solve, that is how to provide extrudates comprising silica which upon impregnation with a metal, such as cobalt, do not tend to form metal-hydro-silicates.

It has now been found that silica extrudates impregnated with a metal selected from group IVb of the Periodic Table can be made by extrusion of silica together with a dissolved group IVb metal compound, without the use of alkali metal compounds. Finely divided silica and a water soluble group IVb compound, especially an alkaline, water soluble zirconium compound, are mulled together in the presence of water, followed by extrusion of the mixture and optionally drying and/or calcination of the extrudates. The silica/group IVb metal oxide extrudates thus obtained show good crush strength and are very suitable for the preparation of Fischer-Tropsch catalysts having a high activity, a high selectivity and a good stability.

The invention therefore relates to a process for the preparation of extrudates suitable for use in the manufacture of catalysts or catalyst carriers, comprising mulling a mixture of finely divided silica, a water soluble group IVb compound and water, which mixture has a solids content of from 20 to 50% by weight, and extruding the mixture.

The finely divided silica to be used in the process of the present invention usually comprises silica particles having an average diameter which is less than 100 »m, preferably between 15 and 80 »m, more preferably between 35 and 65 »m.

The silica which may be used in the process of the invention is often indicated as amorphous silica, and is usually a porous silica. The word amorphous, when used in combination with silica, denotes a lack of crystal structure, as defined by X-ray diffraction. Some short-range ordering may be present and is indicated by electron diffraction studies but this ordering gives no sharp X-ray diffraction pattern. The extent of porosity may be indicated for instance by the pore volume and or the surface area. For general information about amorphous silica, reference is made to Kirk-Othmer, Encyclopedia of Chemical Technology, Third edition, Vol. 20, p. 766 ff.

A suitable silica to be used in the process of the invention is silica gel, a more or less coherent, rigid, continuous three-dimensional network of particles of colloidal silica. The amount of silicium dioxide is usually between 96 and 99.5% by weight. The aggregate particle size is usually between 3 and 25 »m, while the ultimate particle size is usually between 1 and 100 nm. The surface area may vary between 150 and 900 m²/g, and is often between 200 and 700 m²/g. Especially suitable silica gels are spray dried silica gels. It is preferred not to use calcined silica gels, that is silica gels which have been heated to temperatures around 500 °C and higher.

A preferred kind of silica to be used in the process of the present invention is precipitated silica. Precipitated silica is composed of aggregates of ultimate particles of colloidal size that have not become linked in massive gel network during the preparation process. The amount of silicium dioxide is usually between 80 and 99.5 % by weight. The aggregate particle size is usually between 3 and 65 »m, while the ultimate particle size is usually between 3 and 30 nm. The surface area may vary between 30 and 900 m²/g, and is often between 45 and 700 m²/g.

Precipitated silica may be prepared from a silicate solution, preferably a sodium or potassium silicate, using a relatively low silicate concentration when compared with silica gel preparation by addition of an acid, preferably sulphuric acid or hydrochloric acid. The precipitates are separated from the mother liquor by filtration. It is especially preferred in the process of the present invention to use the filter cake which is obtained after filtration of the reaction product as described above, more preferably the washed and/or spray dried filter cake. Washing may be carried out with water, but is preferably carried out with an electrolyte solution having a pH lower than 6. An organic acid, for instance acetic acid, or an inorganic acid, for instance hydrogen fluoride or nitric acid, or salts thereof may be used. Washing may also be carried out after spray drying of the filter cake.

Another preferred silica to be used in the process of the present invention is pyrogenic or fumed silica. This type of silica is usually obtained in high temperature processes as vaporising silica, usually sand, at 2000 °C and cooling, thus forming anhydrous amorphous silica particles. Other processes are the oxidation of silicon tetrachloride vapour with oxygen or with hydrogen and/or methane and flame hydrolysis of silicon ester vapours. Pyrogenic silica tends to be less dense than other types of silica. The amount of silicium dioxide is usually more than 99.5% by weight. The aggregate particle size is usually between 1 and 10 »m, often between 2 and 5 »m, while the ultimate particle size is usually between 1 and 100 nm. The surface area may vary between 10 and 500 m²/g, and is often between 15 and 400 m²/g.

When silica, for instance silica gel, is gradually heated to a higher temperature it loses water. The water content of a silica sample is often determined by weight loss on ignition (LOI). The silica sample is weighed and placed in a furnace where it is heated at 1000 °C for 2 hours. After heating the sample is weighed again, the weight representing the solids content of the sample. The difference in the weights represents the LOI or the amount of water present in the sample. The same procedure may be used for the determination of the LOI of other samples.

The purity of the silica to be used in the process of the present invention is preferably more than 97% by weight based on water free samples, preferably more than 98%, more preferably more than 99%. It is preferred to use a silica which contains an amount of sodium which is less than 10,000 ppmw, more preferably less than 6,000 ppmw, still more preferably less than 2,000 ppmw. The amount of sulphate is suitably less than 7,500 ppmw, preferably less than 4,500 ppmw, more preferably less than 1500 ppmw.

The silica to be used in the process of the present invention may be washed before use in order to improve the purity. Water or an electrolyte solution may be used. The washing solution preferably has a pH lower than 6. Suitable washing solutions are aqueous solutions of organic acids, for instance alkanoic acids having 1 to 12 carbon atoms, preferably 1 to 6 carbon atoms, and dicarboxylic acids, preferably containing 1 to 6 carbon atoms. Very suitably alkanoic acid as formic acid, acetic acid, propionic acid and butyric acid may be used. Acetic acid is especially preferred. Very suitable dicarboxylic acids are oxalic acid, malonic acid, succinic acid, glutaric acid and phthalic acid. The above mentioned acids may be substituted by alkoxy groups, particularly having less than five carbon atoms, hydroxy groups and cyano groups. Beside washing solutions containing organic acids, also washing solutions containing inorganic acids as hydrogen fluoride, hydrogen chloride, hydrogen bromide, nitric acid, nitrous acid and perchloric acid may be used. Further, salts of the before mentioned acids may be used, for instance ammonium salts, or mixtures of the aforementioned acids and one or more salts thereof.

A suitable pore volume of the starting silica particles is between 0.8 and 1.5 ml/g, preferably between 1.15 and 1.35 ml/g. A suitable surface area is between 100 and 500 m²/g, preferably between 200 and 400 m²/g. Suitable pore-diameters are between 8 and 50 nm, preferably between 12 and 30 nm.

The solids content of the mixture to be mulled in the process of the present invention is preferably between 30 and 45% by weight, more preferably about 40%. The amount of water should at least be such that a mix is formed having the proper consistency for extrusion. A suitable volume of aqueous impregnation solution corresponds to the pore volume of the silica to be impregnated plus ten percent.

The water soluble group IVb compound to be used in the process may be any water soluble group IVb compound or mixtures thereof, and is preferably a water soluble zirconium compound. Preferably a group IVb compound is used which gives an alkaline solution on dissolution in water. Suitable group IVb compounds may be salts derived from organic acids, especially acetic acid and propionic acid, but also compounds like acetylacetonate derivatives may be used. Other compounds are group IVb halides, group IVb oxy halides and cyclopentadienyl derivatives, e.g. zirconocene. A preferred compound is ammonium zirconium carbonate, either as such or as a stabilised solution, for instance a solution stabilised by an organic acid as tartaric acid. Also water soluble titanium compounds, especially titanium compounds which on dissolving in water result in basic solutions, may very suitably be used in the process of the present invention. The amount of group IVb metal to be used is conveniently between 3 and 100% by weight based on the amount of group IVb metal dioxide having regard to the amount of water free silica. Preferably the amount of group IVb metal is between 5 and 60%, more preferably between 10 and 40%.

After mixing the components, the mixture is mulled for a certain period, usually between 10 and 120 minutes, preferably between 15 and 90 minutes. The mixing of the components is suitably carried out by mixing the silica and the group IVb compound, followed by addition of water. In a preferred embodiment the group IVb compound is first dissolved in the water, whereafter the solution is added to the silica. The latter procedure results in an extrudate having a very homogeneous distribution of the group IVb compound over the silica. During the mulling process a certain amount of energy is introduced into the mixture. The amount of energy is suitably between 0.05 and 50 Wh/min/kg, preferably between 0.5 and 10 Wh/kg/min. The mulling process is suitably carried out at a temperature between 15 and 50 °C, although lower or higher temperatures may be used. Because of the uptake of energy the temperature at the end of the mulling process will be higher than at the beginning. Any suitable commercially available muller can be employed.

In order to obtain strong extrudates, i.e. extrudates having a high crush strength, especially after drying and/or calcination, it may be preferred to add a certain amount of a basic compound to the mixture. It is especially preferred to add ammonia, an ammonia releasing compound or an organic amine or ammonium compound to the mixture as these compounds do not leave behind any traces after calcination. A preferred organic amine compound is ethanol amine.

The basic compound is preferably added to the mixture after addition of the water, as otherwise a large amount of the basic compound would be taken up into the pores of the silica where it contributes little or not at all to the strength of the extrudates. Thus, the process carried out in the above indicated way needs less basic compound, which makes the process cheaper while less basic compound has to be removed before or during calcination. In the case of the use of basic group IVb metal compounds the amount of basic compound may be diminished, or may be even unnecessary, which is favourable from a technical point of view (less contamination, better product) as well as from an economical point of view.

The amount of basic compound to be added to the mixture may be established by measuring the pH of the mixture. The pH may be measured with a combined glass-electrode by taking equal aliquots of mix and water, and stirring intensively for a minute until a homogeneous slurry is obtained. It is preferred to obtain a mixture having a pH between 8.5 and 11.5, preferably between 9.0 and 11.0.

After the mulling has been carried out, the mixture may be extruded in any conventional extruder. A screw type machine may be used to extrude the mix through a die plate with orifices of the desired form and size. The strands formed upon extrusion, optionally after cutting to the the desired length, are usually dried and optionally calcined. Cylindrical extrudates may be prepared, but other forms may be prepared as well, for instance forms mentioned in US patent 4,028,227. It is preferred to prepare trilobe extrudates. Very suitably hollow cylinders, for example cylinders having a central hollow space which has a radius of between 0.1 to 0.4 of the radius of the cylindrical extrudate, and rifled (or twisted) trilobes may be used. Suitable (nominal) diameters may vary between 0.5 and 5 mm, preferably between 1 and 3 mm.

It has appeared that extrusion of mixtures having a high pH is more difficult than mixtures having a pH in the range of 7.0 to 8.5. Thus, it is preferred in those cases in which the mixture has a pH higher than 8.5 to decrease the pH to a value between 7.5 to 8.5 by addition of an acid compound. Preferably an organic acid is used, as these acids do not leave any traces behind after drying and calcination. Suitable organic acids are for instance alkanoic acids having 1 to 12 carbon atoms, preferably 1 to 6 carbon atoms, and dicarboxylic acids, preferably containing 1 to 6 carbon atoms. Very suitably alkanoic acid as formic acid, acetic acid, propionic acid and butyric acid may be used. Acetic acid is especially preferred. Very suitable dicarboxylic acids are oxalic acid, malonic acid, succinic acid, glutaric acid and phthalic acid. The above mentioned acids may be substituted by alkoxy groups, particularly having less than five carbon atoms, hydroxy groups and cyano groups. Beside organic acids, also inorganic acids as hydrogen fluoride, hydrogen chloride, hydrogen bromide, nitric acid, nitrous acid and perchloric acid may be used.

To improve the flux properties of the mixture in the extruder a surface active agent or a polyelectrolyte may be added to the mixture. The addition of the surface active agent or the polyelectrolyte further results in a smoother extrudate texture and facilitates cutting of the extruded product. Further, formation of macropores in the calcined catalytic material may be improved which may enhance the catalytic properties of these products. As surface active agents may be used cationic surface active agents, for example fatty amines, quaternary ammonium compounds, aliphatic mono- carboxylic acids, ethoxylated alkyl amines, polyvinyl pyridine, sulphoxonium, sulphonium, phosphonium and iodonium compounds, anionic surface active agents, for example alkylated aromatics, acyclic monocarboxylic acids, fatty acids, sulphonated aromatics, alcohol sulphates, ether alcohol sulphates, sulphated fats and oils and phosphonic acid salts, and nonionic surface active agents, for example polyoxyethylene alkylphenols, polyoxyethylene alcohols, polyoxyethylene alkylamines, polyoxyethylene alkylamides, polyols and acetylenic glycols. The amount of flux improver is suitably between 2 and 8 % (w/w), preferably between 3 and 5 % (w/w). A preferred flux improver is sold under the trademark Nalco.

It is possible prior to extrusion to admix with the mixture titanium dioxide, zirconium dioxide and/or aluminium trioxide, or precursor compounds therefor such as hydroxides of titanium, zirconium or aluminium. Other admixtures that may be used are for instance oxides of gallium, indium, thorium, uranium, magnesium and zinc. The amount of each of the added compounds as indicated above is suitably up to 20% by weight calculated on the amount of silica carrier, preferably up to 10%, more preferably up to 5%. The total amount most suitably does not exceed 50% by weight calculated on the amount of silica carrier, preferably does not exceed 30%, more preferably does not exceed 15%.

The silica extrudates obtained according to the process of the present invention as described hereinbefore can be calcined at a temperature between 400 °C and 1000 °C, preferably between 600 °C and 900 °C. Calcination may be carried out in conventional calcination equipment. Very suitably (heated) air or an exhaust gas, obtained for instance by burning liquid or gaseous hydrocarbons, may be used as calcination gas, but other gases, for instance nitrogen, argon and carbon dioxide may also be used.

The silica extrudates, especially after drying and calcination, may be used for the preparation of catalysts, especially catalysts which may be used in hydrocarbon conversion processes like hydrotreatment, hydrocracking and hydrodemetallization of heavy hydrocarbon oils, in hydrocarbon synthesis reactions, in the epoxidation of olefinically unsaturated compounds with organic peroxides, in the hydration of olefinically unsaturated compounds to produce the corresponding alkanols, in the hydrogenation of aromatics compounds, amides, nitriles, cyanides etc., in the dehydration of alcohols and in the purification of exhaust gases.

The extrudates for use in hydrodemetallization are most suitably obtained by the calcination of a zirconium-containing silica extrudate, to which is added molybdenum after the calcination process.

The silica extrudates are preferably used in the preparation of hydrocarbons from synthesis gas, a reaction which is known in the literature as the Fischer-Tropsch reaction. Very suitable Fischer-Tropsch catalysts as well as a very suitable process in which the catalysts may be applied have been described in European patent application No. 127220.

Catalysts to be used for hydrocarbon synthesis may be prepared by application of a suitable, catalytically active metal, preferably cobalt, on the silica extrudates as described hereinbefore. The metal may be applied on the dried and/or calcined extrudates by one or more known techniques, for instance kneading, impregnation, precipitation etc. Preferably impregnation is used. Impregnation may be carried out by contacting a compound of the relevant metal in the presence of a liquid, usually in the form of a solution of the relevant metal compound. As metal compounds organic and inorganic compounds may be used. The liquids used may be organic or inorganic. Mixtures of liquids may also be used. Preferred cobalt compounds are inorganic cobalt compounds, preferably cobalt nitrate. A preferred impregnation liquid is water. In all preparation procedures the liquid used for the metal application is removed from the composition, whereafter the dried composition usually is calcined and optionally reduced. Cobalt is preferably used as catalytically active metal. Other suitable metals which may be used are for instance nickel, iron and ruthenium. Mixtures may also be used. The amount of catalytically active metal to be deposited on the carrier is suitably between 3 and 100 pbw per 100 pbw of silica in the carrier, preferably between 10 and 80 pbw, still more preferably between 20 and 60 pbw. If necessary, one or more metal or metal oxide promoters may also be applied to the extrudates, either before application of the catalytic active metal or after application of the catalytic active metal. The promoter(s) may also be applied before as well as after application of the catalytically active metal. Suitable metal oxide promoters may be chosen from the groups IIa, IIIb, IVb and Vb of the Periodic Table as well as from the actinides and lanthanides. Also chromium may be used. Very suitably magnesium, calcium, strontium, barium, scandium, yttrium, lanthanum, cerium, titanium, zirconium, hafnium, thorium, uranium, vanadium and chromium may be used. Suitable metal promoters may be chosen from group VIIb or VIII of the Periodic Table. Very suitably rhenium and group VIII noble metals (especially ruthenium, platinum and palladium) may be used. The amount of promoter is suitably between 0.1 and 150 pbw per 100 pbw of silica in the carrier.

These catalyst are used in a process for the preparation of hydrocarbons by contacting a mixture of carbon monoxide and hydrogen with a catalyst suitable for the production of hydrocarbons from synthesis gas, which catalyst is prepared according to the processes as described above. Before contacting the catalyst with the hydrogen/carbon monoxide mixture, the catalysts are usually activated by reduction with hydrogen or a hydrogen containing gas. The reduction may very suitably be carried out at a temperature between 200 and 350 °C and a pressure between 2 and 20 bar. The temperature, the total pressure and the hydrogen partial pressure in the mixture may be varied in such a way that an optimal catalyst performance is obtained.

The conversion of the mixture of hydrogen and carbon monoxide may be carried out at a temperature between 125 and 350 °C, preferably between between 175 and 250 °C, and a pressure between 5 and 100 bar, preferably between 12 and 50 bar.

The hydrogen and carbon monoxide containing feed which is to be converted into hydrocarbons by using a catalyst prepared according to the present invention preferably has an H₂/CO molar ratio higher than 1.5, preferably between 1.75 and 2.25. If the feed has an H₂/CO molar ratio lower than 1.5, the latter is preferably raised to have a value between 1.5 and 2.5, preferably between 1.75 and 2.25. It is observed that when non-converted hydrogen and carbon monoxide is recirculated over the catalyst bed, it is possible to choose the circumstances in such a way that the catalyst is contacted with a synthesis gas having a substantially lower H₂/CO ratio than the feed synthesis gas has. Thus, the selectivity to longer hydrocarbon chains may be improved.

The catalysts prepared according to the above described processes when used for the conversion of hydrogen/carbon monoxide mixtures yield a substantially paraffinic product whose high-boiling part may be converted in high yield into middle distillates by the use of a catalytic hydrotreatment. The feed for the hydrotreatment chosen is at least the part of the product whose initial boiling point lies above the final boiling point of the heaviest middle distillates desired as end product, although also the complete product may be used as feed for the catalytic hydrotreatment in order to improve simultaneously the properties of the directly obtained middle distillates (reduction of unsaturated compounds and oxygenates, hydroisomerisation). The catalytic hydrotreatment is carried out by contacting the fraction to be treated at elevated temperature and pressure and in the presence of hydrogen with a catalyst containing one or more metals with hydrogenation activity supported on a carrier. Examples of suitable catalysts are catalysts containing nickel and/or cobalt and in addition molybdenum and/or tungsten supported on a carrier such as alumina or silica-alumina. In the catalytic hydrotreatment it is preferred to use a catalyst containing one or more noble metals from group VIII of the Periodic Table supported on a carrier. The quantity of the noble metal present in the catalyst may vary within wide limits, but is often between 0.05 and 5 %w. The noble metals from group VIII which may be present are platinum, palladium, ruthenium, iridium, osmium or mixtures thereof, platinum being preferred. The quantity of the group VIII metal in the catalyst is preferably 0.1 to 2 %w, and in particular 0.1 to 1%w. Examples of suitable carriers are silica, alumina, magnesia zirconia, zeolites and mixtures thereof, preferably a mixture of silica and alumina. Suitable conditions for carrying out the catalytic hydrotreatment are a temperature of 175 to 400 °C, a hydrogen partial pressure of 10 to 250 bar, a space velocity of 0.1 to 5 kg.l⁻¹.h⁻¹ and a hydrogen/oil ratio of 100 to 5000 Nl.kg⁻¹. The catalytic hydrotreatment is preferably carried out under the following conditions: a temperature of 250 to 350 °C, a hydrogen partial pressure between 25 and 100 bar, a space velocity of 0.25 to 2 kg.l⁻¹.h⁻¹ and a hydrogen/oil ratio of 250 to 2000 Nl.kg⁻¹.

The invention is illustrated by the following examples.

### EXAMPLE 1

A mixture was prepared having the following composition:
silica (silica gel, average particle size 18 »m, pore volume 1.3 cm³/g, surface area 325 m²/g), ammonium zirconium carbonate (14% by weight calculated as ZrO₂ on SiO₂) and water, the mixture having a loss on ignition of 59%. The mixture was mulled for 30 minutes, extruded using a steel dieplate, dried at 120 °C and calcined at 700 °C. Cylindrical extrudates were obtained having a reasonable crush strength.

### EXAMPLE 2

Example 1 was repeated, but after mulling acetic acid was added in such an amount that the pH decreased from about 9.5 to 8.3. After addition of the acetic acid mulling was continued for 10 minutes. Extrusion could now be performed more easily. Cylindrical extrudates were obtained having the same crush strength as in Example 1.

### EXAMPLE 3

Example 2 was repeated using 4% by weight of an polyelectrolyte (Nalco) to improve extrusion. The polyelectrolyte was added after the second mulling operation. After addition mulling was continued for another 10 minutes. Cylindrical extrudates were obtained having the same crush strength as in example 2.

### EXAMPLE 4

Example 3 was repeated. Instead of cylindrical extrudates trilobes were made having a nominal diameter of 1.4 mm. Trilobes with a good crush strength were obtained (0.8 MPa).

### EXAMPLE 5

A mixture was prepared having the following composition: silica (silica gel, 800 g), mono-ethanol amine (40 g) and water (900 g). The mixture was prepared by adding water to the silica, followed by the mono-ethanol amine, whereafter the mixture was kneaded for 45 minutes. A solution of zirconium acetate (440 g containing 22% by weight of zirconium calculated as ZrO₂) was added to the mixture, whereafter the mixture (LOI 60%) was extruded using 3% by weight of polyelectrolyte (Nalco). After drying and calcining at 800 °C trilobed extrudates (diameter 1.7 mm) were obtained having a reasonable crush strength (0.3 MPa).

### EXAMPLE 6

Example 1 was repeated, but using pyrogenic silica (Aerosil 380, surface area 380 m²/g). After mulling a mixture was obtained having a pH of 8.7 and a LOI of 60%, which could very well be extruded. Trilobed extrudates were obtained (effective pore diameter 1.4 mm) having a very sharp pore size distribution and a good crush strength.

### EXAMPLE 7

Example 4 was repeated, but using silica (precipitated silica, average particle size 50 »m, surface area 450 m²/g). Trilobes were obtained with a good crush strength.

### EXAMPLE 8

Example 4 was repeated, but using silica (precipitated silica, average particle size 50 »m, surface area 450 m²/g) which has been washed several times with an ammonium carbonate solution. Trilobes with a good crush strength were obtained.

### EXAMPLE 9

A mixture is prepared from 2105 g of silica powder (average particle size 17 »m, pore volume 1.3 cm³/g, surface area 310 m²/g), 1620 g ammonium zirconium carbonate, 35 g acetic acid, 26.4 g polyelectrolyte (Nalco) and 1496 g water. The mixture is mulled for 30 minutes and extruded using a Delrin dieplate. After drying and calcination (800 °C for one hour) of the trilobed extrudates (effective diameter 1.4 mm) a carrier is obtained having the following properties: crush strength 1.0 MPa, surface area 262 m²/g, pore volume 0.8 ml/g, pore diameter 12.7 nm. Zirconium content 12% by weight calculated on silica.

### EXAMPLE 10

The catalyst carriers prepared in Examples 1 to 9 were used for the preparation of Fischer-Tropsch catalysts by impregnating them with cobalt (25 parts by weight cobalt on 100 pbw silica). Impregnation was carried out using a concentrated solution of cobalt nitrate. After impregnation of the carriers the impregnated particles were dried and calcined. After activation with hydrogen, the activated catalyst particles were contacted in a fixed bed reactor with synthesis gas (H₂/CO ratio 1.1, inlet pressure 29 bar). A heavy wax was obtained. The temperature for 100 STY varied for all the examples between 214 and 234 °C, the C₅+ selectivity varied between 82 and 88%. For instance the carrier prepared in example 4 resulted in a 100 STY temperature of 214 °C and a C₅+ selectivity of 88%. For the carrier prepared in Example 7 these figures are 226 °C and 87.5% respectively.

### EXAMPLE 11

A mixture was prepared having the following composition: silica (precipitated silica, average particle size 50 »m, surface area 450 m²/g), ammonium zirconium carbonate (20% by weight calculated as ZrO₂ on SiO₂) and water, the mixture having a loss on ignition of 73%. The resulting mixture was mulled for 20 minutes. An aqueous solution of acetic acid (70% by weight) was added to give a mixture having a loss on ignition of 72%. The mixture was mulled for a further 10 minutes. Polyelectrolyte (Nalco) (4% by weight) was added and the resulting mixture mulled for a further 5 minutes. The mixture was extruded using a Delrin dieplate. The resulting trilobe extrudates were dried (330 to 350 °C) and calcined (800 °C for one hour). The resulting extrudates had the following properties: crush strengths 0.88 MPa, surface area 371 m²/g, pore volume 1.07 ml/g, pore diameter 19.10 nm and a ZrO₂ content of 13.3% by weight.

### EXAMPLES 12 to 15

The general procedure of Example 11 was repeated a further four times. The properties of the resulting extrudates are given in Table 1.

**Table 1**

| Example | crush strength (MPa) | surface area (m²/g) | pore volume (ml/g) | pore diameter (nm) | zirconium content (%w) |
|---|---|---|---|---|---|
| 12 | 1.01 | 386 | 1.040 | 18.82 | 10.2 |
| 13 | 0.96 | 383 | 1.121 | 18.56 | 10.2 |
| 14 | 0.93 | 381 | 1.118 | 19.75 | 10.1 |
| 15 | >1.3 | 247 | 0.64 | 20.2 | 8.17 |

### EXAMPLE 16

The catalyst carrier prepared in Example 15 was used for the preparation of Fischer-Tropsch catalysts by impregnation with cobalt (21.8% by weight cobalt). Impregnation was carried out using a concentrated solution of cobalt nitrate. After impregnation, the resulting catalyst was dried and calcined. The catalyst particles were packed in a fixed bed, activated by reduction with hydrogen, and contacted with synthesis gas (H₂/CO ratio 1.1, inlet pressure 36 bar). A heavy wax was obtained at a STY of 95 g/l/h and a temperature of 214 °C with a C₅+ selectivity of 89.5%.

### EXAMPLES 17 to 20

Four separate batches of extrudates were prepared using the general procedure of Example 11 from the following components:

| | |
|---|---|
| Precipitated silica (particle size 50 »m; surface area 450 m²/g) | 2257 g |
| Ammonium zirconium carbonate | 1161 g |
| Acetic acid (5%) | 116 g |
| Polyelectrolyte (Nalco) (4%) | 93 g |
| Water | 3620 g. |

The extrudates from each batch were washed in an aqueous solution of ammonium acetate (1 M) for 30 minutes and calcined for a further period of 1 hour at 500 °C. The resulting extrudates were impregnated with cobalt by immersion in an aqueous solution of cobalt nitrate (18% by weight) for 8 hours at 80 °C and then calcined for 2 hours at 500 °C.

To test their performance in Fischer-Tropsch synthesis, catalyst particles from the batches were each loaded into a fixed bed, activated by reduction with hydrogen and contacted with synthesis gas (H₂/CO ratio 1.1, inlet pressure 25 bar). The performance of each catalyst is set out in Table 2 in terms of the temperature necessary to achieve an STY of 100 g/l/h and the C₅+ selectivity.

**Table 2**

| Example | Temperature for 100 STY (°C) | C₅+ selectivity (%w) |
|---|---|---|
| 17 | 216 | 90.5 |
| 18 | 215 | 91.5 |
| 19 | 212 | 91.0 |
| 20 | 220 | 88.0 |

## Claims

1. Process for the preparation of extrudates suitable for use in the manufacture of catalysts or catalyst carriers, comprising mulling a mixture of finely divided silica, which comprises silica particles having an average diameter which is less than 100 »m, a water soluble compound derived from a metal selected from group IVb of the Periodic Table and water, which mixture has a solids content of from 20 to 50% by weight, and extruding the mixture.

2. Process according to claim 1 wherein the finely divided silica comprises particles having an average diameter between 15 and 80 »m, preferably between 35 and 65 »m.

3. Process according to claim 1 or 2, wherein the metal selected from group IVb of the Periodic Table is zirconium.

4. Process according to any one of claims 1 to 3, wherein the finely divided silica is selected from silica gel, precipitated silica and pyrogenic silica.

5. Process according to any one of claims 1 to 4, wherein the silica is washed with an aqueous solution before use.

6. Process according to claim 5, wherein the aqueous solution is an electrolyte solution.

7. Process according to claim 6, wherein the aqueous solution has a pH lower than 6.

8. Process according to any one of claims 3 to 7, wherein the soluble zirconium compound is ammonium zirconium carbonate.

9. Process according to any one of claims 1 to 8, wherein the solid content of the mixture to be extruded is between 30 and 45% by weight, preferably about 40%.

10. Process according to any one of claims 1 to 9, wherein the amount of group IVb metal is between 5 and 60% by weight of the amount of silica.

11. Process according to any one of claims 1 to 10, wherein the pH of the mixture during mulling is between 8.5 and 10.

12. Process according to claim 11 wherein the pH is obtained by addition of ammonia or an organic base.

13. Process according to either of claims 11 or 12, wherein the pH of the mixture is decreased to between 7.0 and 8.5 before extrusion.

14. Process according to claim 13 wherein the pH is decreased by addition of an organic acid.

15. Process according to any one of claims 1 to 14, wherein a surface active agent is added to the mixture to be extruded.

## Patentansprüche

1. Verfahren zur Herstellung von zur Anwendung in der Herstellung von Katalysatoren oder Katalysatorträgern geeigneten Extrudaten, umfasssend ein Mahlen eines Gemisches aus feinverteiltem Siliziumdioxid, das Siliziumdioxidteilchen mit einem mittleren Durchmesser kleiner als 100 »m umfaßt, einer wasserlöslichen Verbindung, die von einem aus der Gruppe IVb des Periodensystems der Elemente ausgewählten Metall abgeleitet ist, und Wasser, welches Gemisch einen Feststoffgehalt von 20 bis 50 Gew.-% aufweist, und ein Extrudieren des Gemisches.

2. Verfahren nach Anspruch 1, worin das feinteilige Siliziumdioxid Teilchen mit einem mittleren Durchmesser zwischen 15 und 80 »m, vorzugsweise zwischen 35 und 65 »m umfaßt.

3. Verfahren nach Anspruch 1 oder 2, worin das aus der Gruppe IVb des Periodensystems der Elemente ausgewählte Metall Zirkonium ist.

4. Verfahren nach einem der Ansprüche 1 bis 3, worin das feinteilige Siliziumdioxid unter Silicagel, gefällter Kieselsäure und pyrogener Kieselsäure ausgewählt wird.

5. Verfahren nach einem der Ansprüche 1 bis 4, worin das Siliziumdioxid mit einer wäßrigen Lösung vor dem Gebrauch gewaschen wird.

6. Verfahren nach Anspruch 5, worin die wäßrige Lösung eine Elektrolytlösung ist.

7. Verfahren nach Anspruch 6, worin die wäßrige Lösung einen pH-Wert unter 6 aufweist.

8. Verfahren nach einem der Ansprüche 3 bis 7, worin die lösliche Zirkoniumverbindung Ammoniumzirkoniumcarbonat ist.

9. Verfahren nach einem der Ansprüche 1 bis 8, worin der Feststoffgehalt des zu extrudierenden Gemisches zwischen 30 und 45 Gew.-%, vorzugsweise etwa 40 % beträgt.

10. Verfahren nach einem der Ansprüche 1 bis 9, worin die Menge an Gruppe IVb-Metall zwischen 5 und 60 Gew.-% der Siliziumdioxidmenge beträgt.

11. Verfahren nach einem der Ansprüche 1 bis 10, worin der pH-Wert des Gemisches während des Vermahlens zwischen 8,5 und 10 beträgt.

12. Verfahren nach Anspruch 11, worin der pH-Wert durch Zusetzen von Ammoniak oder einer organischen Base erhalten wird.

13. Verfahren nach Anspruch 11 oder 12, worin der pH-Wert des Gemisches vor dem Extrudieren auf einen Wert zwischen 7,0 und 8,5 abgesenkt wird.

14. Verfahren nach Anspruch 13, worin der pH-Wert durch Zusetzen einer organischen Säure erniedrigt wird.

15. Verfahren nach einem der Ansprüche 1 bis 14, worin dem zu extrudierenden Gemisch ein grenzflächenaktives Mittel zugesetzt wird.

## Revendications

1. Procédé de préparation d'extrudats utilisables dans la préparation de catalyseurs ou de supports de catalyseurs, selon lequel on malaxe un mélange de silice finement divisée, qui comprend des particules de silice ayant un diamètre moyen inférieur à 100 »m, d'un composé soluble dans l'eau dérivé d'un métal choisi dans le groupe IVb du tableau périodique et d'eau, ce mélange ayant une teneur en matières solides comprise entre 20 et 50 % en poids, et on extrude le mélange.

2. Procédé selon la revendication 1, dans lequel la silice finement divisée comprend des particules ayant un diamètre moyen compris entre 15 et 80 »m, de préférence entre 35 et 65 »m.

3. Procédé selon la revendication 1 ou 2, dans lequel le métal choisi dans le groupe IVb du tableau périodique est le zirconium.

4. Procédé selon l'une quelconque des revendications 1 à 3, dans lequel la silice finement divisée est choisie parmi du gel de silice, de la silice précipitée et de la silice pyrogénée.

5. Procédé selon l'une quelconque des revendications 1 à 4, dans lequel la silice est lavée avec une solution aqueuse avant emploi.

6. Procédé selon la revendication 5, dans lequel la solution aqueuse est une solution d'électrolyte.

7. Procédé selon la revendication 6, dans lequel la solution aqueuse a un pH inférieur à 6.

8. Procédé selon l'une quelconque des revendications 3 à 7, dans lequel le composé soluble du zirconium est du carbonate d'ammonium et de zirconium.

9. Procédé selon l'une quelconque des revendications 1 à 8, dans lequel la teneur en matières solides du mélange à extruder est comprise entre 30 et 45 % en poids, et est de préférence d'environ 40 %.

10. Procédé selon l'une quelconque des revendications 1 à 9, dans lequel la quantité de métal du groupe IVb est comprise entre 5 et 60 % en poids de la quantité de silice.

11. Procédé selon l'une quelconque des revendications 1 à 10, dans lequel le pH du mélange durant le malaxage est compris entre 8,5 et 10.

12. Procédé selon la revendication 11, dans lequel le pH est obtenu par addition d'ammoniac ou d'une base organique.

13. Procédé selon l'une des revendications 11 ou 12, dans lequel le pH du mélange est abaissé à un niveau compris entre 7,0 et 8,5 avant l'extrusion.

14. Procédé selon la revendication 13, dans lequel le pH est abaissé par addition d'un acide organique.

15. Procédé selon l'une quelconque des revendications 1 à 14, dans lequel un agent tensio-actif est ajouté au mélange à extruder.
